Europäisches Patentamt

European Patent Office

Office européen des brevets

(19)

(11) EP 1 139 267 A2

(12) **EUROPEAN PATENT APPLICATION**

(43) Date of publication:
04.10.2001 Bulletin 2001/40

(51) Int Cl.$^7$: **G06F 19/00**, G01N 33/573

(21) Application number: 01302779.2

(22) Date of filing: 26.03.2001

(84) Designated Contracting States:
AT BE CH CY DE DK ES FI FR GB GR IE IT LI LU
MC NL PT SE TR
Designated Extension States:
AL LT LV MK RO SI

(30) Priority: 31.03.2000 US 193717 P

(71) Applicant: Pfizer Products Inc.
Groton, Connecticut 06340 (US)

(72) Inventors:
• Ekins, Sean
Indianapolis, Indiana 46202 (US)
• Gao, Feng
Groton, Connecticut 06340 (US)
• Johnson, Diane Lynn
Groton, Connecticut 06340 (US)
• Kelly, Kevin George
Groton, Connecticut 06340 (US)
• Meyer, Ralph Daniel
Groton, Connecticut 06340 (US)

(74) Representative: Hayles, James Richard et al
Pfizer Limited,
Patents Department,
Ramsgate Road
Sandwich Kent CT13 9NJ (GB)

(54) **Single point interaction screen to predict IC50**

(57) This invention provides an improved computationally derived regression-based method for determining $IC_{50}$ or $EC_{50}$ values for chemical compounds, which predicts potential drug-drug interactions involving cytochrome P450 and other enzymes, transporters, receptors or proteins with active site(s). In addition, this approach predicts affinity for target enzymes, transporters and receptor proteins from a single compound concentration, which will rapidly enable identification of therapeutic use.

EP 1 139 267 A2

## Description

Field of the Invention

This invention relates to an improved method for determining the effect of new chemical entities on biologically active proteins. In particular, this invention relates to an improved method for determining the potential for drug-drug interaction involving cytochrome P450s (CYP) with new chemical entities.

Background of the Invention

Unfavorable drug-drug interactions (DDI) are only responsible for approximately 1-2% of clinically relevant DDI (Fuhr *et al*., 1996), but are still an important factor in determining whether a new chemical entity will successfully make it beyond a drug discovery program to development. In addition, the late discovery of a clinically significant drug-drug interaction is costly in terms of the financial investment in a particular project. Therefore it is important to screen for potential interactions (FDA, 1997; Wrighton and Silber, 1996) early on as well as select the most appropriate *in vivo* studies (FDA, 1998; Tucker, 1992). This rapid determination of clinical viability or fast efficient killing of compounds has been commented on previously (Miwa, 1995). In this regard drug interactions with cytochrome P450s (CYPs) are particularly important (Lin and Lu, 1997), but ultimately avoidable (depending on the therapeutic index), even though drug metabolism is complex and to some extent predictable by careful assessment of structure activity relationships. CYP1A2, CYP2C9, CYP2C19, CYP2D6 and CYP3A4 represent greater than 90% of total hepatic P450 (Shimada *et al*., 1994) and nearly 80% of therapeutic drugs are metabolized by these same enzymes (Smith *et al*., 1998). Interaction with one or more of these enzymes *in vivo* would thus pose a potentially clinically relevant event. In recent years, *in vitro* systems have proved invaluable in predicting the likelihood of DDI (Lin and Lu, 1997) as they allow identification of the CYPs responsible for metabolism as well as determination of the relative contribution to overall elimination of the inhibited pathways (Lin, 1998). Since the number of molecules synthesized by pharmaceutical companies has dramatically increased with the utilization of combinatorial chemistry, there is now a shift in emphasis towards earlier implementation of higher throughput *in vitro* studies for metabolism (Moody *et al*., 1999; Rodrigues, 1997) or lead optimization (Tarbit and Berman, 1998). The prediction of drug-drug interactions of new chemical entities (NCEs) using *in vitro* methods, such as human liver microsomes, hepatocytes (Pichard *et al.,* 1990) or individual expressed CYPs has escalated both in importance and scale of use, as one way to reliably avoid potential interactions *in vivo* (Tucker, 1992). However, occasionally there may be discrepancies when comparing predictions in each system (Lin, 1998) as ultimately metabolism *in vivo* is complicated by the role of transport processes (Ito *et al.*, 1998).

As far as the technologies available for determining DDI *in vitro,* groups are now utilizing multiwell well plates, multiwell pipetting, column switching, automation and mass spectroscopy (MS) (Korfmacher *et al.*, 1997) which are revolutionizing sample throughput for drug metabolism and are easily applicable. A number of groups have looked to radiolabeled substrates as a means of increasing the speed of screening for CYP interactions without the need for high performance liquid chromatography (HPLC) or MS detection (Hopkins *et al.,* 1998; Moody *et al.*, 1999; Rodrigues *et al.*, 1997). However, this technique has the considerable disadvantage of creating low level radioactive waste for disposal, which in some locations may be both costly and undesirable. In contrast, following on from the use of fluorescent probes with whole cells cultured in 96 well plates (Donato *et al.*, 1993), others have looked at fluorescent probes and plate reader technologies with expressed CYPs (Crespi *et al.*, 1997; Crespi *et al.*, 1998). Ultimately, these methods are severely limited due to potential interference of the non optimal spectral characteristics of the fluorescent substrate, requirement for increased levels of expressed enzymes and the frequent observation of activation with some substrates and inhibitors (Mankowski, unpublished observation). There are also some important limitations with *in vitro* incubations which should be considered (Bertz and Granneman, 1997; Ekins *et al.*, 1998a; Ekins *et al.*, 1998b; Maenpaa *et al.*, 1998). Perhaps the most significant is the effect of organic solvents which has been widely evaluated (Busby Jr *et al.*, 1999; Chauret *et al.*, 1998; Hickman *et al.*, 1998), hence the solvent volume added to the incubation is ultimately restricted. We are then left with more efficiently using the multiwell plate, liquid handling and analytical detection technologies already available to us. As the speed and sensitivity of analytical detection using MS has increased, allowing very short run times (less than a minute), this no longer provides a bottleneck.

Another way to optimize the throughput of current CYP interaction screens has already been addressed by some groups, namely the possibility of using fewer inhibitor concentrations in order to fit more determinations on a 96 well plate (Moody *et al.*, 1999; Wynalda and Wienkers, 1997). Although in some cases correlations have been presented to validate this direction (Moody *et al.*, 1999), there has been little or no discussion of the relevance or utility of single point screening for CYP interaction studies. Likewise, there is some indecision as to the number of determinations for each sample e.g. use of duplicates or single points, as well as the number of controls used. In future, the importance of screening for DDI with all CYPs and other enzymes involved in drug metabolism will be high and automation of all liquid handling, incubation and analytical techniques will leave the emphasis on the number of samples obtainable per plate. Beyond the logistics of the experiment itself is the use of techniques such as statistical experimental design, whereby computational approaches are used to determine the nature of inhibition as well as estimating kinetic constants like $K_i$ and $IC_{50}$ (Bronson *et al.,* 1995; Lutz *et al.*, 1996). By determining signal windows for high throughput screens, another group has shown the benefits of using compounds in single wells as opposed to duplicates, which would naturally double the number of compounds on a multiwell plate and positively affect the cost and time of screening (Sittampalam *et al.*, 1997). With these considerations in mind, the present study evaluates 10 or 3 point CYP $IC_{50}$ screening and the potential for using a single point interaction screen to predict $IC_{50}$ for DDI studies. This would be a means of optimizing the present 96 well plate technology to the fullest extent and achieve the goal of these updated CYP interaction screens. Ultimately, high throughput DDI screens will provide data to generate and validate predictive CYP computational models (Ekins *et al.*, 1999a; Ekins *et al.*, 1999b).

Decreasing the time and resources to screen experimental drug compounds results in the ability to screen more compounds for a given investment, and increase the chances of discovering commercially viable compounds. One method for decreasing the time and resources for biological screening is to reduce the number of concentrations used in standard assays. Biological activity, as measured by $IC_{50}$, the concentration of compound that results in 50% of the maximal inhibition, or $EC_{50}$, the concentration that results in 50% of the maximal effect, can be determined by a mathematical relationship teamed after an initial period of screening at multiple concentrations. Screening at a single concentration increases the number of compounds that can be screened, and also reduces the quantity of compound that must be prepared for screening, saving on preparatory resources. For many assays that we have studied, screening at a single concentration results in equivalent precision of the $IC_{50}$ value to that obtained by screening at multiple concentrations.

References

Banks, M., Binnie, A., and Fogarty, S.: High throughput screening using fully integrated robotic screening. *J Biomolecular Screening* **2:**133-135, 1997.

Becker, R. A., Chambers, J. M., and Wilks, A. R.: The S language, Wadsworth and Brooks / Cole Advanced Books & Software, Pacific Grove, CA, 1988.

Bertz, R. J., and Granneman, G. R.: Use of *in vitro* and in vivo data to estimate the likelihood of metabolic pharmacokinetic interactions. *Clin Pharmacokinet* **32:** 210-258, 1997.

Bronson, D. D., Daniels, D. M., Dixon, J. T., Redick, C. C., and Haaland, P. D.: Virtual kinetics: using statistical experimental design for rapid analysis of enzyme inhibitor mechanisms. *Biochem Pharmacol* **50:** 823-831, 1995.

Busby Jr, W. F., Ackermann, J. M., and Crespi, C. L.: Effect of methanol, ethanol, dimethyl sulfoxide, and acetonitrile on in vitro activities of cDNA-expressed human cytochromes P-450. *Drug Metab Dispos* **27:** 246-249, 1999.

Chauret, N., Gauthier, A., and Nicoll-Griffith, D. A.: Effect of common organic solvents on in vitro cytochrome P450-mediated metabolic activities in human liver microsomes. *Drug Metab Dispos* **26:** 1-4, 1998.

Crespi, C. L., Miller, V. P., and Penman, B. W.: Microtiter plate assays for inhibition of human drug-metabolizing cytochromes P450. *Anal Biochem* **248**: 188-190, 1997.

Crespi, C. L., Miller, V. P., and Penman, B. W.: High throughput screening for inhibition of cytochrome P450 metabolism. *Med Chem* Res **8**: 457-471, 1998.

Cronin, M. T.: Computer-aided prediction of drug toxicity in high throughput screening. *Pharm Pharmacol Commun* **4:** 157-163, 1998.

Donato, M. T., Gomez-lechon, M. J., and Castell, J. V.: A microassay for measuring cytochrome P450IA1 and P450IIB1 activities in intact human and rat hepatocytes cultured on 96-well plates. *Anal Biochem* **213:** 29-33, 1993.

Draper, A. J., Madan, A., and Parkinson, A.: Inhibition of coumarin 7-hydroxylase activity in human liver microsomes. *Arch Biochem Biophys* **341:** 47-61, 1997.

Draper, N. R., and Smith, H.: Applied regression analysis, John Wiley and Sons, New York, 1981.

Ekins, S., Bravi, G., Binkley, S., Gillespie, J. S., Ring, B. J., Wikel, J. H., and Wrighton, S. A.: Three dimensional-quantitative structure activity relationship (3D-QSAR) analyses of inhibitors for CYP3A4. *J Pharmacol Exp Ther* **290:** 429-438, 1999a.

Ekins, S., Bravi, G., Ring, B. J., Gillespie, T. A., Gillespie, J. S., VandenBranden, M., Wrighton, S. A., and Wikel, J. H.: Three dimensional-quantitative structure activity relationship (3D-QSAR) analyses of substrates for CYP2B6 *J Pharmacol Exp Ther* **288:** 21-29, 1999b.

Ekins, S., Maenpaa, J., and Wrighton, S. A.: In vitro metabolism: subcellular fractions. *In* Handbook of drug metabolism, ed. by T. F. Woolf, Marcel Dekker, New York, 1999c.

Ekins, S., Ring, B. J., Binkley, S. N., Hall, S. D., and Wrighton, S. A.: Autoactivation and activation of cytochrome P450s. *Int J Clin Pharmacol Ther* **36**: 642-651, 1998.

FDA: Guidance for industry. Drug metabolism/drug interaction studies in the drug sevelopment process: studies in vitro. , 1997.

FDA: Guidance for industry. In vivo drug metabolism/drug interaction studies - study design, data analysis, and recommendations for dosing and labeling. , 1998.

Fuhr, U., Weiss, M., Kroemer, H. K., Neugebauer, G., Rameis, H., Weber, W., and Woodcock, B. G.: Systematic screening for pharmacokinetic interactions during drug development. *Int J Clin Pharmacol Thera* **34:** 139-151, 1996.

Hickman, D., Wang, J.-P., Wang, Y., and Unadkat, J. D.: Evaluation of the selectivity of in vitro probes and suitability of organic solvents for the measurement of human cytochrome P450 monooxygenase activities. *Drug Metab Dispos* **26:** 207-215, 1998.

Hook, D.: Ultra high-throughput screening - a journey into nanoland with Gulliver and Alice. *Drug Discovery Today* **1:** 267-268, 1996.

Hopkins, A., Howells, K. B., Price-Jones, M. J., Hughes, K. T., Eddershaw, P. J., Hood, S. R., Woodroofe, A. J., and Tarbit, M. H.: A high throughput screening assay for CYP3A4 interactions using SPA. The 12th International Symposium on Microsmes and Drug Oxidations, July 1998, Montpellier, France, 1998.

Ito, K., Iwatsubo, T., Kanimitsu, S., Ueda, K., Suzuki, H., and Sugiyama, Y.: Prediction of pharmacokinetic alterations caused by drug-drug interactions: metabolic interaction in the liver. *Pharmacol Rev* **50:** 387-411, 1998.

Korfmacher, W. A., Cox, K. A., Bryant, M. S., Veals, J., Ng, K., Watkins, R., and Lin, C.-C.: HPLC-API/MS/MS: a powerful tool for integrating drug metabolism into the drug discovery process. *Drug Discovery Today* **12:** 532-537, 1997.

Kronbach, T., Mathys, D., Gut, J., Catin, T., and Meyer, U.: high performance liquid chromatography assays for bufuralol 1'-hydroxylase, debrisoquine 4-hydroxylase and dextromethorphan O-demethylase in microsomes and purified cytochrome P-450 isozymes of human liver. *Anal Biochem* **162:** 24-32, 1987.

Leeman, T., Transon, C., and Dayer, P.: Cytochrome P450TB (CYP2C): A major monooxygenase catalyzing diclofenac 4'-hydroxylation in human liver. *Life Sci* **52:** 29-34,1992.

Lin, J. H., and Lu, A. Y. H.: Inhibition of cytochrome P-450 and implications in drug development. *Ann Rep Med Chem* **32:** 295-304, 1997.

Lin, J. H. a. L., A.Y.H.: Inhibition and induction of cytochrome P450 and the clinical implications. *Clin Pharmacokinet* **35:** 361-390, 1998.

Lutz, M. W., Menius, J. A., Choi, T. D., Laskody, R. G., Domanico, P. L., Goetz, A. S., and Saussy, D. L.: Experimental design for high-throughput screening. *Drug Discovery Today* **1:** 277-286, 1996.

Maenpaa, J., Hall., S. D., Ring, B. J., Strom, S. C., and Wrighton, S. A.: Human cytochrome P4503A (CYP3A) mediated midazolam metabolism: the effect of assay conditions and regioselective stimulation by a-napthoflavone, terfenadine and testosterone. *Pharmacogenetics* **8,** 1998.

Mankowski, D.I., Elsenboss, L., Christopherson, P., Lawton, M.P and Tweedie, D.J: Characterizationof baculovirus-expressed human cytochromes P450. 11th International Symposium on microsomes and Drug oxidations P55 (1996).

Miwa, G. T.: Goals, design, timing, and future opportunities for non clinical drug metabolism studies. *Toxicol Pathol* **23:**131-135, 1995.

Moody, G. C., Griffin, S. J., Mather, A. N., McGinnity, D. F., and Riley, R. J.: Fully automated analysis of activities catalysed by the major human liver cytochrome P450 (CYP) enzymes: assessment of human CYP inhibition potential. *Xenobiotica* **29:** 53-75,1999.

Pichard, L., Fabre, I., Fabre, G., Domergue, J., Aubert, B. S., Mourad, G., and Maurel, P.: Screening for inducers and inhibitors of cytochrome P-450 (cyclosporin A oxidase) in primary cultures of human hepatocytes and in liver microsomes. *Drug Metab Dispos* **18:** 595-606, 1990.

Rodrigues, A. D.: Preclinical drug metabolism in the age of high-throughput screening: An industrial perspective. *Pharm Res* **14**:1504-1510,1997.

Rodrigues, A. D., Surber, B. W., Yao, Y., Wong, S. L., and Roberts, E. M.: [O-ethyl 14C] phenacetin O-deethylase activityin human liver microsomes. *Drug Metab Dispos* **25**:1097-1100,1997.

Shimada, T., Yamazaki, H., Mimura, M., Inui, Y., and Guengerich, F. P.: Interindividual variations in human liver cytochrome P-450 enzymes involved in the oxidation of drugs, carcinogens and toxic chemicals: studies with liver microsomes of 30 Japanese and 30 Caucasians. *J Pharmacol Exp Pharmacol* **270:** 414-423, 1994.

Singh, J., Soloweij, J., Allen, M., Killar, L., and Ator, M.: Lead development: validation and application of high throughput screening for determination of pharmcokinetic parameters for enzyme inhibitors. *Bioorg Med Chem* **4:** 639-643, 1996.

Sittampalam, G. S., Iversen, P. W., Boadt, J. A., Kahl, S. D., Bright, S., Zock, J. M., Janzen, W. P., and Lister, M. D.: Design of signal windows in high throughput screening assays for drug discovery. *J Biomolecular Screening* **2:** 159-169, 1997.

Smith, D. A., Abel, S. M., Hyland, R., and Jones, B. C.: Human cytochrome P450s: selectivity and measurement in vivo. *Xenobiotica* **28:** 1095-1128, 1998.

Sonderfan, A. J., Arlotto, M. P., Dutton, D. R., McMillen, S. K., and Parkinson, A.: Regulation of testosterone hydroxylation by rat liver microsomal cytochrome-P450. *Arch Biochem Biophys* **255:** 27-41, 1987.

Spencer, R. W.: High-throughput screening of historic collections: observations on file size, biological targets and file diversity. *Biotechnol Bioeng* **61:** 61-67, 1998.

Tarbit, M. H., and Berman, J.: High-throughput approaches for evaluating absorption, distribution, metabolism and excretion properties of lead compounds. *Curr Opin Chem Biol* **2:** 411-416, 1998.

Tucker, G. T.: The rational selection of drug interaction studies: implications of recent advances in drug metabolism. *Int J Clin Pharmacol Thera Toxicol* **30:** 550-553, 1992.

van der Hoeven, T. A., and Coon, M.: Preparation and properties of partially purified cytochrome P-450 and reduced nicotinamide adenine dinucleotide phosphatecytochrome P450-reductase from rabbit liver microsomes. *J Biol Chem* **249:** 6302-6310, 1974.

van der Voet, H.: Comparing the predictive accuracy of models using a simple randomization test. *Chem Int Lab Systems* **25:** 313-323, 1994.

Wrighton, S. A., Ring, B. J., and VandenBranden, M.: The use of in vitro metabolism techniques in the planning and interpretation of drug safety studies. *Toxicologic Pathology* **23** (2): 199-208, 1995.

Wrighton, S. A., and Silber, P. M.: Screening studies for metabolism and toxicology. *In* Scientific strategies for accelerated drug discovery, University of Wisconsin Press, Wisconsin, 1996.

Wrighton, S. A., Vandenbranden, M., Stevens, J. C., and Shipley, L. A.: In vitro methods for assessing human hepatic drug metabolism: their use in drug development. *Drug Metab Rev* **25** (4): 453-84, 1993.

Wynalda, M. A., and Wienkers, L. C.: Assessment of potential interactions between dopamine agonists and various human cytochrome P450 enzymes using a simple in vitro inhibition screen. *Drug Metab Dispos* **25:**1211-1214, 1997.

Summary of the Invention

This invention provides a method for routine determination of the $IC_{50}$ values for compounds via biological assay at a single concentration.

The term "$IC_{50}$" means the concentration of a test compound which produces 50% of the maximal inhibition on a biologically active protein target. "$IC_{50}$" as used herein includes "$EC_{50}$", the concentration of a test compound which produces 50% of the maximal effect on a biologically active protein target. As used herein, the term "target" means a biologically active protein. Targets include cytochromes P450, enzymes, receptors, and transporters.

This invention provides a method for routine determination of $IC_{50}$ values for compounds via biological assay at a single concentration, which comprises:

a.) Developing or identifying a biological assay capable of producing a percent inhibition or percent effect for a compound tested at a known concentration;

b.) Performing the assay on an initial collection of at least 10 compounds, and at least 1 commercially available compound to be used as positive control, eachassayed at a set of 3 to 10 or more concentrations, measuring a percent inhibition or percent effect at each concentration for each compound;

c.) Determining an $IC_{50}$ for each of these initial compounds and the positive control compound(s), by fitting a mathematical dose response curve, such as the Hill function,

$$percent\ inhibition = \frac{100}{1 + \left(\dfrac{IC_{50}}{concentration}\right)^{h}}$$

to the data for each compound, using a computer, and standard linear or nonlinear regression techniques;

d) Using the resultant data from these initial compounds to fit a mathematical relationship between the $IC_{50}$ values and the percent inhibition values at a single fixed concentration X,

$IC_{50}$= f(percent inhibition) in general, or, for example,

$IC_{50}$= exp{a + b •(percent inhibition at concentration *X*)},

e) Using a computer, and standard linear or nonlinear regression techniques, resulting in an equation relating $IC_{50}$ to percent inhibition or percent response on all remaining and future test compounds, at the previously fixed single concentration X, and determining the $IC_{50}$ via the mathematical equation developed in step d).

This invention further provides a method for determining $IC_{50}$ values for drug-drug interactions related to cytochrome P450 (CYP).

This invention further provides a method wherein said CYP is CYP2C9.

This invention further provides a method wherein said CYP is CYP2D6.

This invention further provides a method wherein said CYP is CYP3A4.

This invention further provides a method wherein X is 3.

This invention further provides a method wherein X is greater or less than 3.

This invention further provides a method wherein said CYP is CYP1A2.

This invention further provides a method wherein said CYP is CYP2C19.

This invention further provides a method wherein said CYP is recombinant CYP2D6.

This invention further provides a method wherein said CYP is replaced by any enzyme.

This invention further provides a method wherein said CYP is replaced by any human, mammalian, plant, fungal, bacterial or insect derived enzyme.

This invention further provides a method wherein said CYP is replaced by any human, mammalian, plant, fungal, bacterial or insect derived transporter.

This invention further provides a method wherein said CYP is replaced by any human, mammalian, plant, fungal, bacterial or insect derived receptor.

This invention further provides a method wherein said CYP is produced by molecular biology techniques and expressed in human, animal, insect, fungal, bacterial, yeast or viral cells.

This invention further provides a method wherein said CYP is replaced by any human, mammalian, plant, insect, fungal, yeast, bacterial or viral derived enzyme, transporter or receptor produced by molecular biology techniques and expressed in human, animal, insect, fungal, yeast, bacterial or viral cells.

Brief Description of the Drawings

Figure 1. Comparison of 10 point and 3 point $IC_{50}$ [ M] for sample proprietary compound data. For experimental details see Examples.

Figure 2. Relationship between $\log_{10}(IC_{50})$ [ M] and percent inhibition at 3 M for proprietary compound data. For experimental details see Examples.

Figure 3. Comparison of positive control compounds and proprietary compound data. For experimental details see Examples.

Figure 4. Regression models of $\log_{10}(IC_{50})$ [ M] vs. percent inhibition at 1, 3 or 10 M. For experimental details see Examples.

Figure 5. Reference distribution of the randomized T based on 1000 numbers generated from the Monte Carlo simulations under the null hypothesis, and the observed T value.

Figure 6. One point predicted $IC_{50}$ [ M] using percent inhibition at 3 M vs. 10 or 3 point $IC_{50}$ [ M] on the test set. For experimental details see Examples.

Figure 7. Regression model with percent inhibition at 3 M and 95% prediction interval and all but four sample proprietary compound data for the three screenings. For experimental details see Examples.

Figure 8. Regression model for positive control compounds. For experimental details see Examples.

Figure 9. Regression model with percent inhibition at 3 $\mu$M for CYP1A2 data. For experimental details see Examples.

Figure 10. Regression model with percent inhibition at 3$\mu$M for CYP2C9 and CYP2C19 data. For experimental details see Examples.

Figure 11. Regression model with percent inhibition at 3$\mu$M for recombinant CYP2D6 data. For experimental details see Examples.

Figure 12. Final regression model with percent inhibition at 3 $\mu$M for all CYP data. For experimental details see Examples.

Detailed Description of the Invention

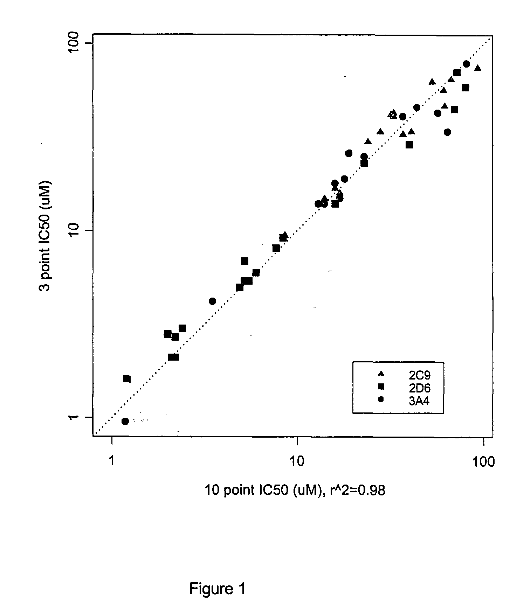

Drug-drug interactions involving cytochrome P450 (CYP) are an important factor in whether a new chemical entity will survive through to the development stage. Therefore, the identification of this potential as early as possible *in vitro,* saves considerable future unnecessary investment. *In vitro* CYP interaction screening data for CYP2C9, CYP2D6 and CYP3A4 was analyzed to determine the correlation of 10 and 3 point determinations ($r^2$ = 0.98, Figure 1). Following this we investigate whether a single point could also be predictive of $IC_{50}$. We found that the $IC_{50}$ value could be predicted by a single value of percent inhibition at either 10, 3 or 1 M. This enables determination of more $IC_{50}$ values on a multi-well plate and results in more economical use of compounds. Statistical analysis of proprietary compound data for CYP2C9, CYP2D6 and CYP3A4 showed that there is a strong linear relationship between $\log_{10}$

($IC_{50}$) and percent inhibition at 3 M ($r^2 = 0.90$) and that it is possible to predict a compound's $IC_{50}$ value by the percent inhibition value obtained at 3 M. The 95% prediction boundary for this is roughly 0.3 on $log_{10}$ scale which is comparable to the variability of *in vitro* determinations for positive control $IC_{50}$ data (Table 1 in Example 8). More data (for CYP2C19, CYP1A2 and recombinant CYP2D6) were obtained which enabled the model to be updated. The final model is described in detail below. The use of a single inhibitor concentration would offer the opportunity to drastically speed up screening for CYP interactions, which is important with the challenges provided by combinatorial chemistry generating orders of magnitude more new chemical entities. In addition, this algorithmic approach would obviously be applicable for other *in vitro* bioactivity and therapeutic target enzyme screens that have historically utilized multiple compound concentrations to determine $IC_{50}$ or $EC_{50}$ values.

Initially, $IC_{50}$ values for 204 data points from CYP screen CYP2C9, CYP2D6 and CYP3A4 were available. Amongst the 204 data points, 163 were from proprietary compounds, and 41 were from commercially available compounds that were used as positive controls. The $IC_{50}$ values were generated based on percent inhibition at either 10 or 3 different concentrations. The 10 point $IC_{50}$ values were compared with 3 point $IC_{50}$ values and a high correlation was observed ($r^2=0.98$, Figure 1). This naturally let us to investigate whether we could reliably predict $IC_{50}$ using fewer than 3 points, i.e. a single point screen. The 10 point $IC_{50}$ or 3 point $IC_{50}$ were typically generated through the fit of a dose-response curve of a particular functional format. In the CYP screening, the dose-response curve used is the well-known Hill function which can be expressed as:

$$(100 - \textit{percent inhibition at x}) = \frac{100}{1 + (IC_{50}/x)^{h}},$$

where $x$ is the concentration and $h$ is the Hill parameter, which is set to -1 here. In cases like this, there is usually a close correlation between the $IC_{50}$ value and percent inhibition at a fixed concentration. For example, if the dose-response function used is the above mentioned Hill function, then it is possible to show that

$$IC_{50} = \frac{x \cdot (\textit{percent inhibition at x})^{1/h}}{(100 - \textit{percent inhibition at x})^{1/h}}$$

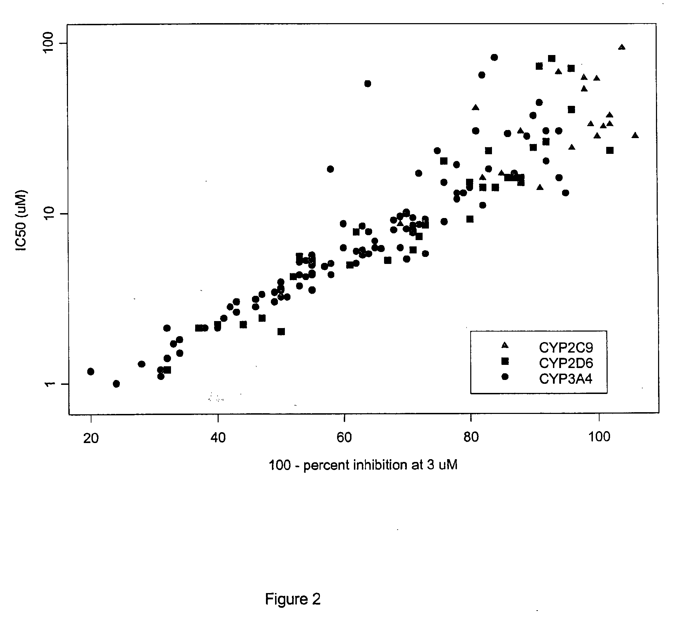

Therefore it is also possible to find a high correlation between $log_{10}(IC_{50})$ and percent inhibition at 3 μM (Figure 2). The variation seen in the plot (data does not all fall on a thin curve) is caused by factors such as measurement error and variations caused using different human liver microsome lots. This type of data can be analyzed by a statistical method and a mathematical model can then be built that describes the relationship between the variables (in this case, $IC_{50}$ and percent inhibition at concentration x) as well as the variations in this data. We used regression analysis to analyze the data and build the mathematical model. Our analysis was carried out using the statistical software Splus (Becker *et al.,* 1988). Regression analysis was performed as described previously (Draper and Smith, 1981). Details of the analysis can be found in Example 3.

During the analysis, we needed to decide whether to use percent inhibition at 1 μM or percent inhibition at 3 μM or percent inhibition at 10 μM in the model. To do this we used a randomization t-test proposed by H. van der Voet (van der Voet, 1994) to compare the predictive nature of the three models. The result of the randomization t-test helped us to decide to use percent inhibition at 3 μM in the model. Further details of this procedure are described in Example 3. In general, statistical model selection procedures, such as the one just mentioned above, can be used to help select the appropriate model.

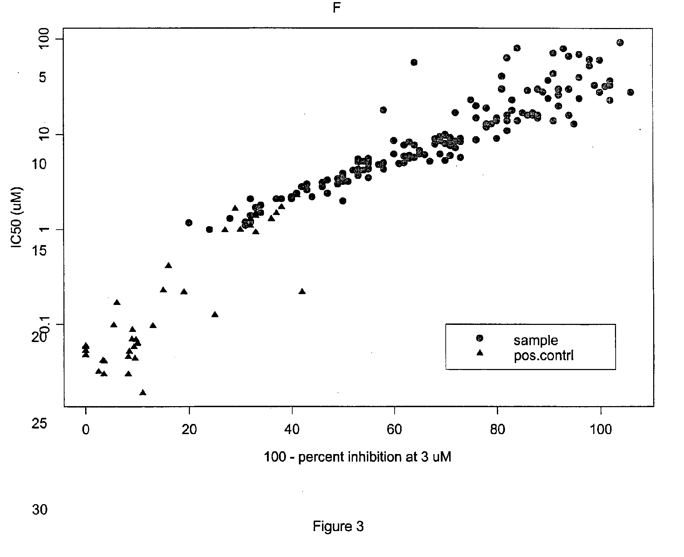

An examination of all the initially available data (Figure 3) shows that the positive control compounds all fall in the low $IC_{50}$ and low 100-percent inhibition at 3 M region, and they mostly do not overlap with the proprietary compound data. In addition the relationship between $log_{10}(IC_{50})$ and 100-percent inhibition at 3 M seems to follow a different slope for positive control compounds. This is particularly evident with $IC_{50}$ values less than 0.5μM. Therefore separate models were produced for proprietary compounds and the positive control compounds. For the positive control compounds, the best model was produced by using percent inhibition at 1 μM as the independent variable. The slope is different from that for proprietary compounds. Details of this are also given in Example 3.

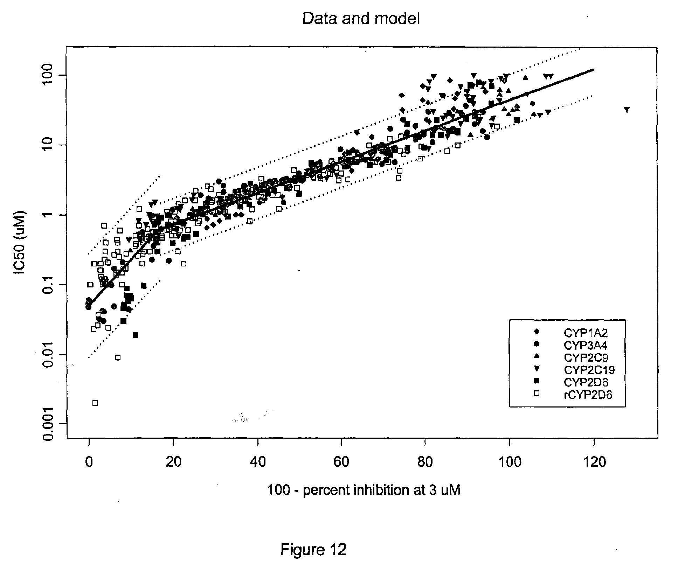

Since the initial analysis, more data for CYP screening, such as CYP2C19 and CYP1A2 and recombinant CYP2D6 (rCYP2D6) were obtained. The new data were added to the initial data and more analysis was performed (see Examples 4, 5, 6). After combining all the available data, we found that there is very little difference among the models for individual CYP screens. We therefore decided to build one model for CYP1A2, CYP2C9, CYP2C19, CYP2D6, rCYP2D6 and CYP3A4 screens (Example 7). We used a regression model and the percent inhibition at 3 μM as the independent variable in the model. As mentioned before, the data suggest that there should be at least two different slopes, one for very potent compounds and one for less potent compounds. We used a statistical method to determine how many different slopes there should be and where the change point should be. For the CYP screen data, we found that two different slopes with a change point at (100-percent inhibition at 3 $\mu$M) = 17 would yield the smallest residual mean squared error. Therefore the model has two different slopes with a change point at (100-percent inhibition at 3 $\mu$M) = 17. Details are in Example 7 and Figure 12.

Examples

**Materials.** Quinidine, Isocitric dehydrogenase, DL-isocitric acid, NADP, ticlopidine, acetophenetidin, and diclofenac were purchased from Sigma Chemical Co. (St Louis, MO), Ketoconazole, ($\pm$)bufuralol, sulphaphenazole, furafylline and (S)-(+)-mephenytoin were obtained from Gentest Inc (Woburn, MA). 4-Androsten-17$\beta$-OL-3-one was obtained from Steraloids. Magnesium chloride, sodium phosphate monobasic and sodium phosphate dibasic were obtained from Fisher Scientific. All other compounds designated were synthesized at Pfizer (Groton, CT). Solvents were obtained from J T Baker (Phillipsburg, NJ).

**Liver specimens and expressed enzymes.** Human livers were obtained from the following organizations under protocols approved by the appropriate committee for the conduct of human research; SRI International (Menlo Park, CA), International Institute for the Advancement of Medicine (IIAM, Exton, PA), Vitron Inc., (Tucson, AZ), Anatomical Gift Foundation (AGF, Woodbine, GA) and National Disease Research Institute (NDRI, Philadelphia). Microsomes were then prepared using differential centrifugation (van der Hoeven and Coon, 1974). Baculovirus expressed CYP2D6 was produced at Pfizer as described by Mankowski et al. 1996.

Example 1

**IC$_{50}$ determinations in 96 well format - 3 and 10 point screening.** Each of the eight rows in a standard 96-well plate was essentially a separate inhibition curve (for 10 point screening) or 2 inhibition curves (for 3 point screening). First, separate plates were prepared for the substrate and for dilutions of inhibitor. The contents of these two plates were then combined in a 1:1 ratio to make a master plate of substrate and inhibitor solutions (S/I plate). The remaining assay ingredients, a combination of microsomes, (10 %) NADPH generating cofactor solution: (stock solution: 125 mM MgCl$_2$, 0.54 mM NADP, 6.2 mM DL-isocitric acid, 0.5 U/ml isocitric dehydrogenase) and buffer (100 mM sodium phosphate, pH 7.4), were prepared on ice and transferred to a polyvinyl reaction plate (RXN plate). Preparation of these plates required the use Soken 96-well pipettor (Apricot Designs Inc, Encino, CA) and Robbins 96-well pipettor (Robbins Scientific Corporation, Sunnyvale, CA). The RXN plate was preincubated to 37 C using a MJ Research Model PTC-100 automated thermal controller and the reaction is initiated by addition of an aliquot from the S/I plate. The reaction was allowed to proceed at 37 C before being terminated using methanol (10 I). HPLC or mass spec analysis is preceded by filtration of (150$\mu$l) using a Millipore multiscreen-MAHA mixed cellulose esters, triton-free, non-sterile plate.

Example 2

**Phenacetin O-deethylation IC$_{50}$ assay (CYP1A2).** Human liver microsomes (0.5 mg/ml protein), phenacetin (50 $\mu$M) and proprietary inhibitors were incubated, terminated and filtered as described above (Example 1). Furafylline was used as a positive control.

**Diclofenac 4'-hydroxylase IC$_{50}$ assay (CYP2C9).** Human liver microsomes (0.1 mg/ml), diclofenac (10 M) and proprietary inhibitors were incubated, terminated and filtered as described above (Example 1). Sulfaphenazole was used as a positive control in place of proprietary compounds.

**(S)-(+)-Mephenytoin hydroxylase IC$_{50}$ assay (CYP2C19).** Human liver microsomes (0.1 $\mu$M P450), S+ mephenytoin (50 $\mu$M) and proprietary inhibitors were incubated, terminated and filtered as described above (Example 1). Ticlopidine was used as a positive control.

**Bufuralol 1'-hydroxylase IC$_{50}$ assay (CYP2D6).** Human liver microsomes (0.1 $\mu$M P450), bufuralol (10 M) and proprietary inhibitors were incubated, terminated and filtered as described above (Example 1). Quinidine was included as a positive control. Alternatively, recombinant CYP2D6 (0.1 mg/ml), bufuralol (3.4 M), proprietary inhibitor (0.1-10 M) and sodium phosphate (100 mM, pH 7.4) in a total volume of 0.5 ml were preincubated at 37$^\circ$C before addition of NADPH (1 mg/ml) and incubated further. The reactions were then terminated and filtered as described previously before analysis (Example 1).

**Testosterone 6 -hydroxylase IC$_{50}$ assay (CYP3A4).** Human liver microsomes (0.1 $\mu$M P450), testosterone (50 M) and proprietary inhibitors were incubated, terminated and filtered as described above (Example 1). Ketoconazole was included as a positive control.

Example 3

**Regression models for human liver microsomal CYP2C9, CYP2D6 and CYP3A4.** Initially, $IC_{50}$ values for 163 proprietary compounds (run multiple times) were generated using the 10 point curve procedure then compared with values produced using the 3 point curve (r = 0.99, Figure 1). This naturally led us to investigate whether we could reliably predict $IC_{50}$ using fewer than 3 points, i.e. a single point screen. At an inhibitor concentration of 3 M a strong correlation was observed between the $\log_{10}(IC_{50})$ and 100-percent inhibition for the compounds analyzed ($r^2 = 0.90$, Figure 2). CYP2C9, CYP2D6 and CYP3A4 models all follow the same trend at this concentration, in that a linear relationship was observed. Similar linear relationships were also demonstrated for $\log_{10}(IC_{50})$ and 100-percent inhibition at 1 M and at 10 M. It is predictable that there should be a correlation between $\log_{10}(IC_{50})$ and 100-percent inhibition at 3 M, because if the Hill function describes the dose-response relationship well, then $\log_{10}(IC_{50})$ can be expressed as: $\log_{10}(IC_{50})=\log_{10}(3)+(1/h)(\log_{10}$(percent inhibition at 3 M) - $\log_{10}$(100-percent inhibition at 3 M)).

Since (100-percent inhibition at 3 M) and $\log_{10}$(percent inhibition at 3 M)-$\log_{10}$(100-percent inhibition at 3 M) are almost linearly correlated between 20% and 80%, then using (100-percent inhibition at 3 M) as a predictor in a linear model to predict $\log_{10}(IC_{50})$ would make a useful model. Regression analysis of $IC_{50}$ data determined from 10 or 3 inhibitor concentrations was then performed to obtain a prediction model together with the associated uncertainties of the predictions in each case. All of the initial data at 3 M for $\log_{10}(IC_{50})$ against 100-percent inhibition is shown in Figure 3. This figure shows that the positive control compounds all fall in the low $IC_{50}$ and low 100-percent inhibition at 3 M region (Figure 3). In addition, they mostly do not overlap with the proprietary compounds data and the relationship between $\log_{10}(IC_{50})$ and 100-percent inhibition at 3 M seems to follow a different slope for positive control compounds. This is particularly evident with $IC_{50}$ values less than 0.5 $\mu$M. Therefore separate models were produced for proprietary compounds and the positive control compounds.

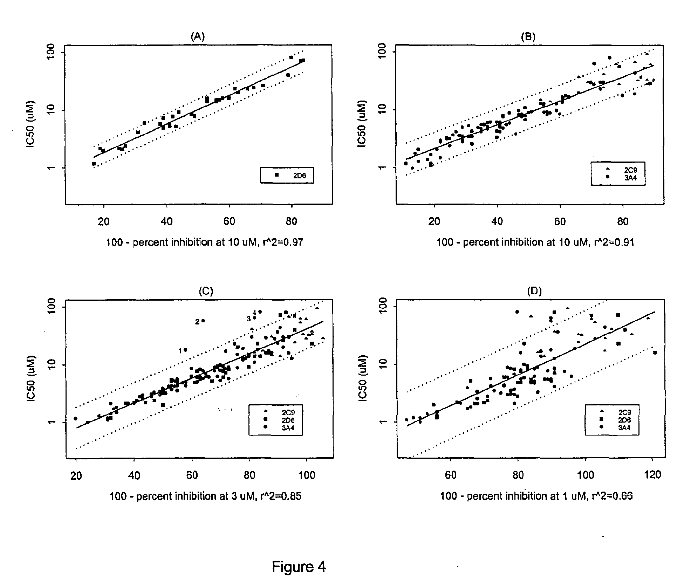

Regression analysis of $\log_{10}(IC_{50})$ vs. 100-percent inhibition at 10 M showed that data from CYP2D6 screens follow a statistically different line than data from the other 2 screens, so CYP2D6 data was fitted to a different model. This is in contrast to the regression analysis of $\log_{10}(IC_{50})$ against 100-percent inhibition at 3 M which showed that data from all 3 screens followed the same line. Regression analysis of $\log_{10}(IC_{50})$ vs. 100-percent inhibition at 1 M showed that we should use data from all the 3 screens to build one model. Figure 4 presents all of these regression models along with the data and the 95% prediction intervals for comparison.

There are three potential models capable of generating one point $IC_{50}$ predictions. Namely, the models using percent inhibition at 1 M, 3 M or 10 M. To test whether there were significant differences in their abilities to predict $IC_{50}$, we used data from all the three screens to fit a single regression for the model using percent inhibition at 10 M. This model turned out to be very similar to the model using data from the CYP2C9 and CYP3A4 screens. We then used a randomization t-test proposed by H. van der Voet (van der Voet, 1994) to compare the predictive nature of the three models. We first compared models with percent inhibition at 10 $\mu$M and with percent inhibition at 3 M. To perform the test we randomly selected 103 data points from the 163 available data points as a training set, while using the remaining 60 data points as a test set. Models were then built using the training set and were followed by predicting the $\log_{10}(IC_{50})$ of the test set. The prediction errors were then calculated for both models. A null hypothesis ($H_0$) was that the squared prediction errors from the two models have the same probability distribution. The alternative hypothesis ($H_1$) used was that the mean squared prediction error from the model with percent inhibition at 3 M was larger than the mean squared prediction error from the model with percent inhibition at 10 M. The differences between the squared prediction errors between the two models were then calculated using the following equation: $d_i = e.3^2{}_i - e.10^2{}_i.$, where $i$ is the index for data in the test set and $e.3_i$ and $e.10_i$ represent prediction errors from the model with percent inhibition at 3 M and the model with percent inhibition at 10 M, respectively. The observed statistic was calculated by: $T_{obs} = $ mean($d_i$) over the test set. A Monte Carlo procedure was then used to simulate the reference distribution of the statistic T under the null hypothesis. We randomly assigned signs to $d_i$ and then calculated the randomized T by: T = mean (signed $d_i$) over the test set.

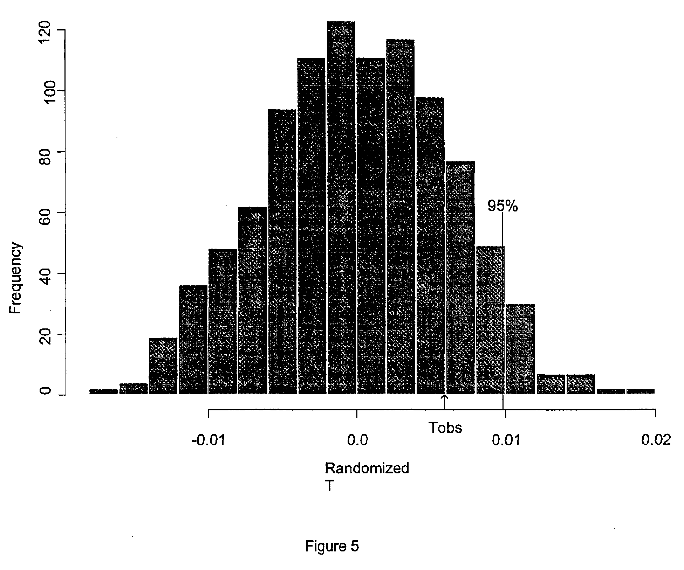

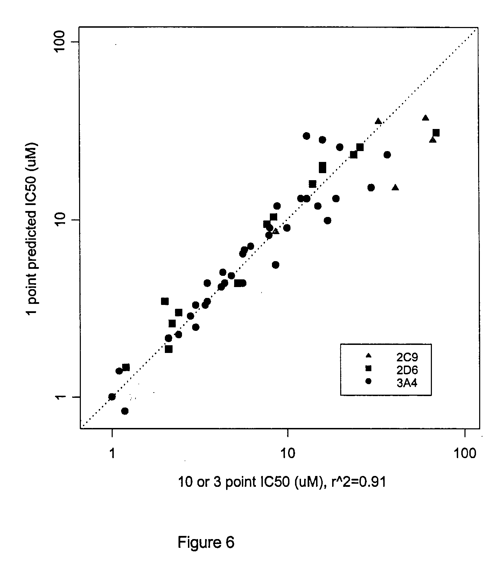

The above steps were repeated 999 times, each time generating a randomized T. These T's provide a simulated distribution of the T under the null hypothesis. If the null hypothesis is true, the observed $T_{obs}$ should be in the "fat" part of the reference distribution of the T's. If the alternative hypothesis is true, the $T_{obs}$ would be somewhere in the upper tail of the distribution of T's. We ranked the $T_{obs}$ among the T's, and found that $T_{obs}$ was ranked 55 from top. Therefore the p-value for this test is 55/1000=0.055, indicating that the difference between the two models is at most marginally significant. If we look at the models and the data (Figure 4C) more closely, we suspect that the bigger mean squared prediction error for model with percent inhibition at 3 $\mu$M might be largely due to the four points marked in the figure. We investigated these points and concluded that the values for these four points were questionable based on the observation of each individual $IC_{50}$ plot. These four points were therefore removed from our data set and the above procedure repeated to determine the model difference statistic. The new p-value was 0.176, indicating that there was no difference between the two models in terms of their ability to predict $IC_{50}$. Figure 5 shows the reference distribution of the randomized T based on 1000 numbers generated from the Monte Carlo simulations under the null hypothesis, and the observed T value. So on the whole the model with percent inhibition at 3 M and the model with percent inhibition at 10 M predicted $IC_{50}$ values equally well. Figure 6 visually compares the predicted $\log_{10}(IC_{50})$ from the regression model with percent inhibition at 3 M (1 point predicted $\log_{10}(IC_{50})$) and the $\log_{10}(IC_{50})$ values for proprietary compounds in the test set which were determined by either 10 point curve or 3 point curve (10 or 3 point $\log_{10}(IC_{50})$). This shows that the model with percent inhibition at 3 M predicts the $IC_{50}$ value well with a single concentration. We then used the same procedure to compare models with percent inhibition at 10 M and with percent inhibition at 1 M. The test concluded that there is a statistically significant difference between the two models in terms of their ability to predict $IC_{50}$ (p-value of 0.001).

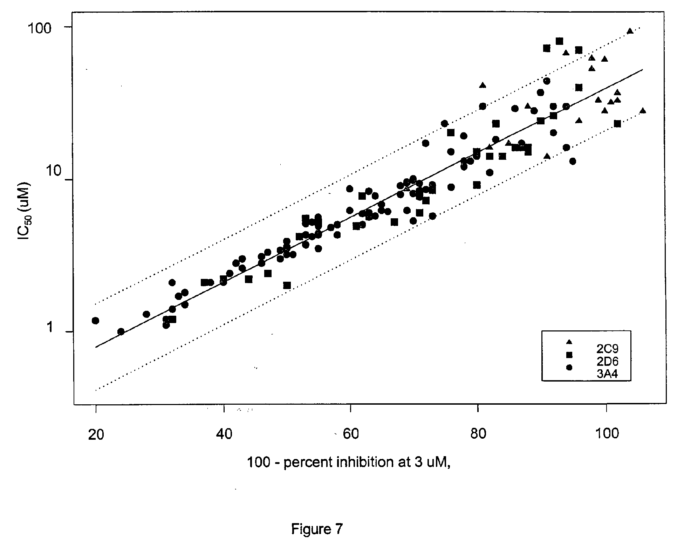

Least squares criterion was used to fit the regression model. The model with percent inhibition at 10 M for CYP2D6 is: predicted $\log_{10}(IC_{50})$ = -0.2238 + 0.0245 $\times$ (100-percent inhibition at 10 M). This resulted in an $r^2$ value of 0.97, p < 0.00001 and residual standard error s = 0.09. The 95% prediction interval is roughly: predicted $\log_{10}(IC_{50})$ 2 $\times$ 0.09. The model with percent inhibition at 10 M for CYP2C9 and CYP3A4 is: predicted $\log_{10}(IC_{50})$ = -0.0778 + 0.0206 $\times$ (100-percent inhibition at 10 M). This resulted in an $r^2$ value of 0.91, p < 0.00001 and s = 0.14. The 95% prediction interval is approximately: predicted $\log_{10}(IC_{50})$ 2 $\times$ 0.14. The model with percent inhibition at 3 M for all three screens is: predicted $\log_{10}(IC_{50})$ = -0.5249 + 0.0212 $\times$ (100-percent inhibition at 3 M). This resulted in an $r^2$ value of 0.90, p < 0.00001 and s = 0.14. The 95% prediction interval is approximately: predicted $\log_{10}(IC_{50})$ 2 $\times$ 0.14. This model together with the data and 95% prediction interval is shown in Figure 7.

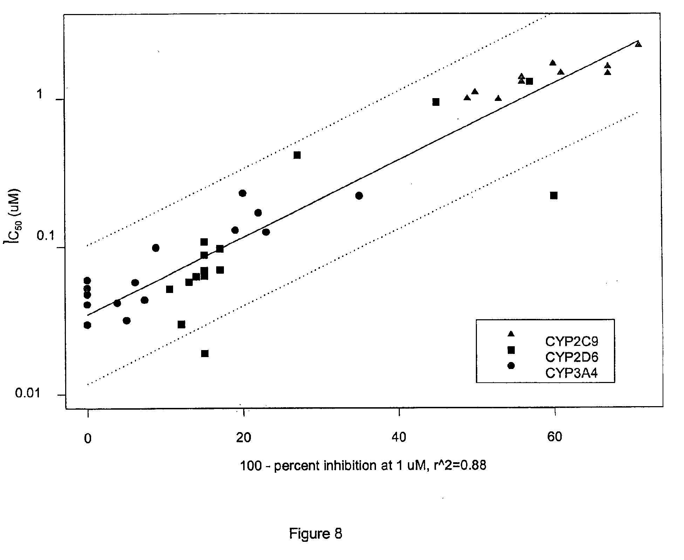

As observed in Figure 3, the slope for $\log_{10}(IC_{50})$ vs. 100-percent inhibition at 3 $\mu$M for positive control compounds appears to be different from that for the sample proprietary compounds with $IC_{50}$ values less than 0.1$\mu$M. Therefore, a separate regression model was generated for positive control compounds. In this case forty data points were available for positive control compounds (Figure 8), where the slope of this line is steeper than that for sample proprietary compound data. As with sample proprietary compounds, least squares criterion was used to fit the model and no difference was found among the three screens. Understandably, due to the fact that all the positive control compounds are very potent, the best model in this case is the one with percent inhibition at 1 M. The equation is: predicted $\log_{10}(IC_{50})$ = -1.4585 + 0.0260 $\times$ (100-percent inhibition at 1 M ), this resulted in an $r^2$ = 0.88, p < 0.00001 and s = 0.23.

Example 4

Using the same mathematical and statistical techniques described in Example 3, and letting x represent (100-percent inhibition at 3 M), 54 data points from CYP1A2 resulted in the following equation:

$$\log_{10}(IC_{50}) = -0.8146 + 0.0277 \cdot x, \; for \; x \geq 19$$

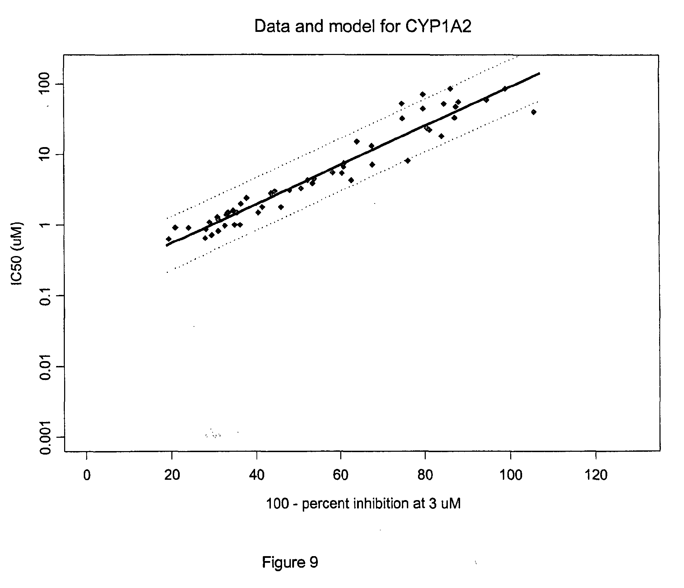

with $R^2$=0.93, residual standard error s=0.18 and p-value for the regression p<0.0001. Figure 9 shows the data and the model. The dotted lines in the figure represent the 95% prediction intervals.

Example 5

Using the same mathematical and statistical techniques described in Example 3, 37 data points from CYP2C19 were analyzed and found to have an identical equation to that of the CYP2C9 data. Using the same notation as in Example 4, the combined data sets of CYP2C19 and CYP2C9 yields the equation:

$$\log_{10}(IC_{50}) = -0.5124 + 0.0219 \cdot x, \; for \; x \geq 9$$

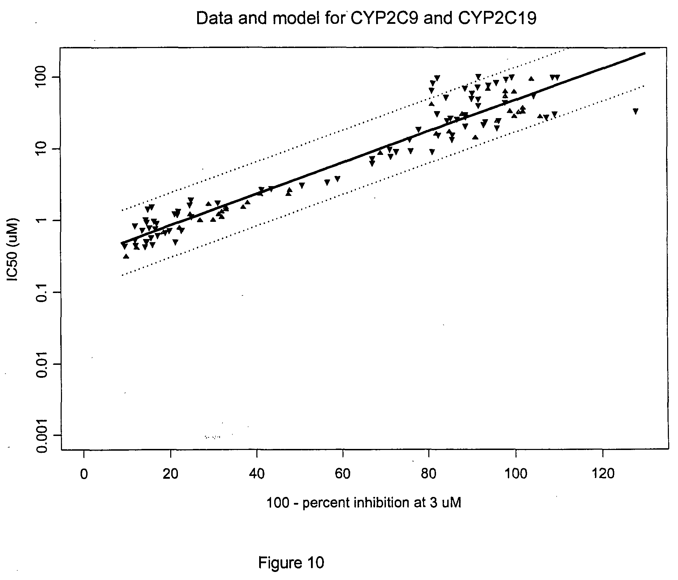

with $R^2$=0.92, s=0.23, n=117, and p<0.0001. Figure 10 shows the data and the model. The dotted lines in the figure represent the 95% prediction intervals.

Example 6

Using the same mathematical and statistical techniques described in Example 3, and using the same notation as in Examples 4 and 5, 175 data points resulted in the following equations which demonstrated for recombinant CYP2D6:

$$\begin{cases} \log_{10}(IC_{50}) = -1.1605 + 0.0572 \cdot x, & for \ x < 18 \\ \log_{10}(IC_{50}) = -0.4603 + 0.0183 \cdot x, & for \ x \geq 18 \end{cases}$$

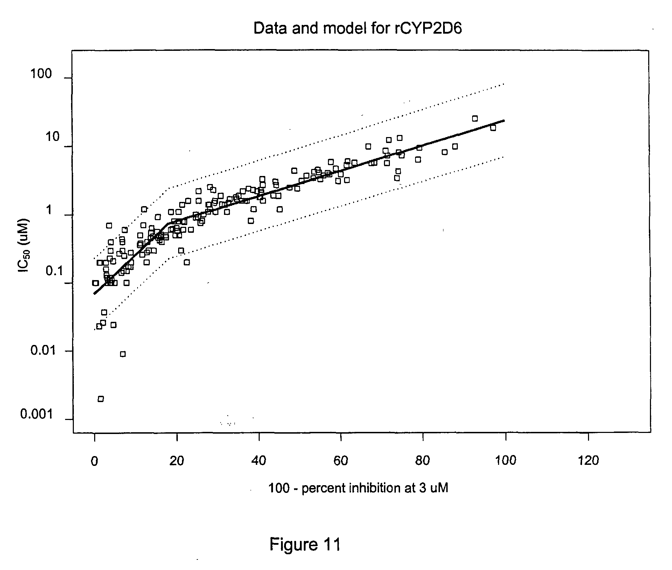

with $R^2=0.85$, s=0.26, n=175, and p<0.0001. Two equations were used to describe the slopes for the potent and less potent inhibitors. Figure 11 shows the data and the model. The dotted lines in the figure represent the 95% prediction intervals.

Example 7

**Combined single point regression model for determining CYP inhibition** By combining the data for CYP1A2, CYP2C19 and rCYP2D6 with the existing data from CYP2C9, CYP2D6 and CYP3A4 screens, we can update the regression model for single point $IC_{50}$ estimation for all the drug-drug interaction CYP screens, including rCYP2D6. This yields a total of 569 valid data points. The data suggests a different slope for very potent compounds (Figure 12), many of which are positive control compounds. The cutoff point for the two slopes is at (100 - percent inhibition at 3 µM) = 17, which was determined statistically as the point that yielded the best result with the smallest residual. This point also corresponds to $IC_{50}$ values around 0.5 - 0.8 µM. Using the notation $x$ to represent the quantity of (100 - percent inhibition at 3 µM), we can write the new regression models for single point $IC_{50}$ estimation potent and less potent compounds as:

$$\begin{cases} \log_{10}(IC_{50}) = -1.2919 + 0.0642 \cdot x, & for \ x < 17 \\ \log_{10}(IC_{50}) = -0.5779 + 0.0222 \cdot x, & for \ x \geq 17 \end{cases}$$

The equation for x < 17 is based on 122 data points. For this model, $R^2=0.46$, the residual standard error s=0.371, and the p-value < 0.0001. The equation for x ≥ 17 is based on 447 data points. For this model, $R^2=0.90$, the residual standard error s=0.187, and the p-value < 0.0001.

Example 8

**Assessment of analytical variability for measuring $IC_{50}$ values** CYP isoform selective inhibitors were used as positive controls to monitor the variability of the method over time. The inhibitor positive controls, furafylline, sulfaphenazole, ticlopidine, quinidine and ketoconazole, were analyzed for CYP1A2, CYP2C9, CYP2C19, CYP2D6 and CYP3A4, respectively. Data were then collected on different days (n 8 inhibition curves). Mean $IC_{50}$ values for furafylline, sulfaphenazole, ticlopidine, quinidine and ketoconazole were 1.195, 0.876, 0.996, 0.093 and 0.051 M, respectively. The inter-assay precision for furalylline, sulfaphenazole, ticlopidine, quinidine and ketoconazole was 33%, 26.1%, 40.1%, 25.7% and 41.2% respectively (Table 1). In contrast, the regression model with percent inhibition at 3 M for most proprietary compounds has a prediction standard error of s=0.187 on $\log_{10}$ scale, which translates into a relative standard deviation (RSD) of roughly log(10) s on the original scale for $IC_{50}$. So we have: RSD for predicting $IC_{50}$ = 2.302 × 0.187 = 43%.

Table 1.

| | CYP1A2 | CYP2C9 | CYP2C19 | CYP2D6 | CYP3A4 |
|---|---|---|---|---|---|
| | Summary of inhibitor positive control data. | | | | |
| | Furafylline | Sulfaphenazole | Ticlopidine | Quinidine | Ketoconazole |
| | $IC_{50}$[ M] | $IC_{50}$[ M] | $IC_{50}$[ M] | $IC_{50}$[ M] | $IC_{50}$[ M] |
| | n=15 | n=30 | n=19 | n=40 | n=45 |
| MEAN | 1.195 | 0.876 | 0.996 | 0.093 | 0.051 |
| S.D. | 0.394 | 0.229 | 0.40 | 0.024 | 0.021 |
| Precision (%) | 33 | 26.1 | 40.1 | 25.7 | 41.2 |

**Claims**

1. A method for routine determination of $IC_{50}$ or $EC_{50}$ values for compounds via biological assay at a single concentration, which comprises:

   a) Identifying a biological assay capable of producing a percent effect for a compound tested for activity against a target at a known concentration;
   b) Performing the assay on an initial collection of at least 10 compounds, and at least 1 commercially available compound to be used as positive control, each assayed at a set of 3 to 10 or more concentrations, measuring a percent effect at each concentration for each compound;
   c) Determining an $IC_{50}$ or $EC_{50}$ for each of these initial compounds by fitting a mathematical dose response curve to the data for each compound, using a computer, and standard linear or nonlinear regression techniques;
   d) Using the resultant data from these initial compounds to fit a mathematical relationship between the $IC_{50}$ or $EC_{50}$ values and the percent inhibition values at a single fixed concentration $X,$
   e) Using a computer, and standard linear or nonlinear regression techniques, developing an equation relating $IC_{50}$ or $EC_{50}$ to percent inhibition or percent response on all remaining and future test compounds, at the previously fixed single concentration $X,$ and determining the $IC_{50}$ or $EC_{50}$ via the mathematical equation developed in step d).

2. The method of claim 1 wherein said mathematical dose response curve is the Hill function,

$$percent\ inhibition = \frac{100}{1 + \left(\dfrac{IC_{50}}{concentration}\right)^{h}}$$

3. The method of claim 1 wherein said mathematical relationship is $IC_{50} = \exp\{a + b \cdot (\text{percent inhibition at concentration } X)\}$.

4. The method of claim 1 wherein said biological assay is an assay for drug-drug interactions related to the target cytochrome P450 (CYP).

5. The method of claim 4 wherein the target is selected from the group consisting of CYP2C9, CYP2D6, CYP3A4, CYP1A2, and CYP2C19.

6. The method of claim 1 where the target is an enzyme.

7. The method of claim 1 wherein the target is a receptor.

8. The method of claim 1 wherein the target is a transporter.

9. The method of claim 1 wherein said biological assay relates to affinity for any target protein wherein modulation of activity is therapeutically desired.

10. The method of claim 1 wherein said biological assay relates to affinity for any nontherapeutic protein wherein modulation of said activity is undesirable.

Figure 1

Figure 2

Figure 3

Figure 4

Figure 5

Figure 6

Figure 7

Figure 8

Data and model for CYP1A2

Figure 9

Figure 10

Data and model for rCYP2D6

Figure 11

Figure 12